Europäisches Patentamt

European Patent Office

Office européen des brevets

(11) Veröffentlichungsnummer: **0 287 004**
**A2**

## (12) EUROPÄISCHE PATENTANMELDUNG

(21) Anmeldenummer: 88105694.9

(22) Anmeldetag: 09.04.88

(51) Int. Cl.⁴ **C07C 69/653 , C07C 67/30**

(30) Priorität: 15.04.87 DE 3712816

(43) Veröffentlichungstag der Anmeldung:
19.10.88 Patentblatt 88/42

(84) Benannte Vertragsstaaten:
BE DE FR GB IT NL

(71) Anmelder: HOECHST AKTIENGESELLSCHAFT
Postfach 80 03 20
D-6230 Frankfurt am Main 80(DE)

(72) Erfinder: Blickle, Peter, Dr.
Hattersheimer Strasse 14
D-6233 Kelkheim (Taunus)(DE)
Erfinder: Hintzer, Klaus, Dr.
Hubertusstrasse 3
D-8269 Burgkirchen(DE)
Erfinder: Schwertfeger, Werner, Dr.
Erlenstrasse 8
D-6306 Langgöns(DE)
Erfinder: Ulmschneider, Dieter, Dr.
Im Flemetz 6
D-6240 Königstein/Taunus(DE)

(54) Verfahren zur Herstellung von Trifluoracrylsäureestern.

(57) Trifluoracrylsäurealkylester wurde hergestellt durch Umsetzung von Hexafluorpropen mit Halogen-fluorosulfat (Halogen X: Cl, Br, J), Spaltung des Fluorosulfates $CF_3-CFX-CF_2-OSO_2F$ zum Säure-fluorid $CF_3-CFX-COF$, Veresterung des Säurefluorids und Enthalogenierung mit einer Metallkomponente.

EP 0 287 004 A2

## Verfahren zur Herstellung von Trifluoracrylsäureestern

Die Erfindung betrifft die Herstellung von Trifluoracrylsäureestern durch ein mehrstufiges Verfahren mit Hexafluorpropen als Ausgangsmaterial.

Ester der Trifluoracrylsäure ($CF_2 = CF-COOH$) sind einsetzbar zur Herstellung von Copolymeren, die -COOR-Seitengruppen enthalten. Diese Copolymeren können beispielsweise nach Umwandlung der Estergruppen in die freie Säure oder in die Salzform als Ionenaustauscherharze oder -membranen Einsatz finden.

Die Herstellung solcher Trifluoracrylsäureester ist schon mehrfach beschrieben worden, jedoch sind die Verfahrensweisen umständlich, die Ausgangsstoffe teuer und die Ausbeuten unbefriedigend.

Eine weitere Veröffentlichung beschreibt ein demgegenüber verbessertes vierstufiges Verfahren (EP-A 0 157 039), das vom Perfluorallylfluorsulfat ausgeht und über Bromanlagerung, Entsulfonierung, Veresterung und Bromabspaltung mit Zink zu den gewünschten Trifluoracrylsäureestern führt. Jedoch besitzt das Ausgangsmaterial, bedingt durch die Herstellung stets ein Beiprodukt, dessen Entfernung einen erheblichen Aufwand erfordert. Aus diesem Grund wird in der Veröffentlichung auch keine Ausbeutebestimmung für den Verfahrensschritt der ersten Stufe angegeben. Alle weiteren Verfahrensstufen, bei denen Halogen angelagert oder abgespalten wird, führen Brom als Halogen an. Die Abspaltung von Fluorhalogen zur Erzielung einer Doppelbindung wird nicht beschrieben. Es was daher erwünscht, eine weitere Verfahrensverbesserung und -vereinfachung zu erreichen.

Die Erfindung betrifft ein vereinfachtes mehrstufiges Verfahren zur Herstellung von Trifluoracrylsäureestern der Formel $CF_2 = CF-COOR$ (I), in der R eine verzweigte oder unverzweigte, unsubstituierte oder mit Halogen substituierte Alkylgruppe mit 1 bis 6 C-Atomen darstellt, bei dem

a) Hexafluorpropen mit einem Halogenfluorosulfat der Formel $X-O-SO_2-F$, in der X Chlor, Brom oder Jod ist, zu $CF_3-CFX-CF_2-OSO_2F$ (II) umgesetzt,

b) aus (II) durch Abspaltung von $SO_2F_2$ das Säurefluorid $CF_3-CFX-COF$ (III) hergestellt,

c) die Verbindung (III) mit einem geradkettigen oder verzweigten unsubstituierten oder halogensubstituierten aliphatischen Alkohol ROH mit 1 bis 6, vorzugsweise 1 bis 4 C-Atomen im Alkylrest R oder mit einem solchen Alkohol, in dem mindestens ein Wasserstoffatom der Alkylkette mit Ausnahme der α-ständigen H-Atome durch Halogen ersetzt ist, zu $CF_3-CFX-COOR$ (IV) verestert und

d) der erhaltene Ester (IV) mit einem Metall in einem aprotischen, polaren Lösungsmittel unter Ausbildung einer Doppelbindung zu Verbindungen der Formel (I) enthalogeniert wird.

Die einzelnen Verfahrensstufen sind z. T. aus der Literatur bekannt, jedoch ist ihre Kombination noch nicht beschrieben worden. Neu und unerwartet ist es, daß die Temperaturen, die bei der Stufe d) zur Abspaltung von Gemischthalogen, d.h. Halogen und Fluor, wobei Halogen Chlor, Brom oder Jod bedeutet und wobei das Fluoratom aus der entständigen $CF_3$-Gruppe abgespalten wird, angewandt werden, bei 60 bis 150°C, vorzugsweise 80 bis 140°C liegen. Bekannt ist zwar die Abspaltung von Gemischthalogen, wie oben definiert, aus Halogen-Fluor-Etherestern bei Temperaturen von 100 bis 300°C, jedoch wird hier das Halogen aus einer entständigen $HalCF_2$-Gruppe abgespalten (DE-A-2934194). Im einzigen Beispiel, in dem ein einstufiges Verfahren zur Herstellung der Fluormetallorganischen Verbindung und deren thermische Zersetzung in einem Lösungsmittel beschrieben wird, wird die Reaktionstemperatur mit 160°C angegeben.

Vorteilhaft ist es bei dem erfindungsgemäßen Verfahren auch, daß bei keiner Verfahrensstufe eine besondere oder aufwendige Reinigung von Zwischenprodukten nötig ist. Etwa vorhandene Nebenprodukte stören nicht oder können leicht abgetrennt werden.

Das zur Durchführung des erfindungsgemäßen Verfahrens benötigte Halogenfluorsulfat $X-OSO_2F$ wird beispielsweise dadurch herstellt, daß man Fluorsulfonsäure in Gegenwart eines Leitsalzes elektrolysiert und die entstandene Lösung von $FSO_2-O-O-SO_2F$ in $FSO_3H$ mit der equimolaren Menge an Chlor, Brom oder Jod umsetzt. Die Reaktion von $FSO_2-O-O-SO_2F$ mit dem betreffenden Halogen kann auch nach Isolation des Peroxids erfolgen.

Bei dem erfindungsgemäßen Verfahren setzt man zuerst in Stufe a) Halogenfluorosulfat mit Hexafluorpropen um und isoliert das Additionsprodukt der allgemeinen Formel II $CF_3-CFX-CF_2-OSO_2F$ (II), das gewöhnlich eine geringe Menge des Bisfluorosulfates $CF_3-CF(OSO_2F)-CF_2-OSO_2F$ enthält. Diese Verunreinigung ist nicht kritisch, da sie als solche oder in Form von Folgeprodukten in den nachfolgenden Umsetzungen leicht abgetrennt werden kann.

In der Verfahrensstufe b) wird das Fluorosulfat (II) in das Säurefluorid $CF_3-CFX-COF$ (III) überführt, wobei nucleophile Verbindung wie Fluoride in Form von löslichen Alkali-und/oder Erdalkalifluoriden, beispielsweise von Natrium, Kalium, Ammonium, Rubidium, Cäsium, Magnesium oder Trialkylamin mit 1 bis 6, vorzugsweise 1 bis 4 C-Atomen in den Alkylresten wie Triethyl-und Tributylamin allein oder in Mischungen als Katalysatoren dienen. In der sich anschließenden Veresterung c) wird mindestens ein aliphatischer gradkettiger oder verzweigter Alkohol ROH mit 1 bis 6, vorzugsweise 1 bis 4 C-Atomen oder solche Alkohole eingesetzt, in denen mindestens ein Wasserstoffatom der Alkylkette mit Ausnahme der α-ständigen H-Atome durch Halogen, vorzugsweise Fluor ersetzt ist, wobei der entstehende Fluorwasserstoff z. B. mit einem Trialkylamin gebunden werden kann. Beispiele für die verwendeten Alkohole sind Methanol, Ethanol, die verschiedenen Propanole und Butanole, ferner 2,2,2-Trifluorethanol, 2,2,3,3-Tetrafluorpropanol, 2,2,3,4,4,5,5-Octafluorpentanol und 1,1,1,3,3,3-Hexafluorpropan-2-ol.

Die Stufen b) und c) können nach einer bevorzugten Ausführungsform des Verfahrens gemeinsam durchgeführt werden, d. h. die Verbindung (II) wird in Gegenwart der vorstehend genannten Katalysatoren mit einem der angegebenen Alkohole umgesetzt.

Die Enthalogenierung der Ester der allgemeinen Formel (IV) zu den Trifluoracylsäureestern der allgemeinen Formel $CF_2=CF-COOR$ (I) wird im allgemeinen mit einem Metall wie Mg, Cu, Sn, vorzugsweise aber mit Hilfe von Zink in einem aprotisch polaren Lösungsmittel wie Polyethylenglykoldimethylether oder Nitrilen, vorzugsweise der Di-oder Tetramethylether des Diethylenglykols (Diglyme oder Tetraglyme) sowie Benzonitril, durchgeführt. Das Zink kann als aktiviertes Zink oder als käuflicher Zinkstaub eingesetzt werden. Eine Aktivierung dieses Zinkstaubs kann durch Ultraschall, schnell laufende Rührer oder auch chemisch durch den Fachmann bekannte Verfahren erfolgen.

Die Erfindung betrifft auch Tetrafluorpropionsäurefluoralkylester der allgemeinen Formel

$CF_3-CFX-COO-CHRR'$

in der X = Cl, Br, J und R und R' unabhängig voneinander Wasserstoff oder Per-bzw. Polyfluoralkyl mit 1 - 4 C-Atomen im Alkylrest bedeuten, jedoch R und R' nicht gleichzeitig Wasserstoff sind, sowie Trifluoracrylsäure-1.1.1.3.3.3-hexafluorisopropylester, $CF_2=CF-COO-CH(CF_3)_2$.

In den nachfolgenden Beispielen bedeutet T Gewichtsteile und % Gewichtsprozent.

**Beispiele:**

1) Darstellung von $CF_3-CFJ-CF_2-OSO_2F$

Zu einer Lösung von 634 T Jod in 1 l fluoroschwefelsäure wurden unter Kühlen 495 T Peroxidisulfuryldifluorid getropft. Die tiefgrüne Lösung wurde noch 5 Stunden bei 25°C gerührt, so daß sie keinen festen Bodensatz mehr aufwies. In diese Lösung wurde durch eine Glasfritte unter kräftigem Rühren Hexafluorpropen (HFP) eingegast. Die Temperatur der Lösung wurde durch Kühlen und durch angepaßte HFP-Einleitung auf ca. 35°C gehalten. Nach starker Aufhellung der Mischung wurde die HFP-Einleitung gestoppt (755 T HFP verbraucht) und noch 1 Stunde gerührt. Das Produkt wurde unter vermindertem Druck abdestilliert, mit Wasser gewaschen und über $Na_2SO_4$ getrocknet. Ausbeute 1691 T (90 % d. Th. bez. auf $JOSO_2F$) Kp. 60°C/135 mbar.

2) Darstellung von $CF_3-CFBr-CF_2-OSO_2F$

Zu 2 l einer 1,47 molaren Lösung von Peroxidisulfuryldifluorid in Fluoroschwefelsäure wurden 470 T Brom getropft. In diese Lösung wurde durch eine Glasfritte unter kräftigem Rühren Hexafluorpropen (HFP) eingeleitet. Die Temperatur wurde dabei durch Kühlen und durch angepaßte HFP-Einleitung auf ca. 40°C gehalten. Gegen Ende der Reaktion hellte sich die Mischung stark auf, nahm jedoch noch weiteres HFP auf, bis dann das Ende der Reaktion durch ausbleibende Wärmeentwicklung angezeigt wurde (HFP-Verbrauch 920 T). Die Mischung bildete zwei Phasen, deren untere (1750 T) als Produktphase abgetrennt wurde. Die obere Phase wurde auf Eis getropft, wonach sich noch eine organische Phase (230 T) abtrennen ließ. Die vereinigten organischen Phasen ergaben nach Destillation 1605 T Produkt (83 % d. Th. bez. auf $BrOSO_2F$); Kp. 93°C.

3) 2-Jodtetrafluorpropionsäurefluorid $CF_3-CFJ-COF$

In einem Glaskolben, der mit Magnetrührer, Thermometer, Tropftrichter, Rückflußkühler und nachgeschalteter Kältefalle (-78°C) ausgestattet war, wurden 10 T Kaliumfluorid und 75 T Diglyme vorgelegt. Das Ansatz wurde auf ca. 50°C erhitzt und 1128 T $CF_3-CFJ-OSO_2F$ so zugetropft, daß eine kontinuierliche Gasentwicklung eintrat. Nachdem die Reaktion beendet war, wurde die Kältefalle auf Raumtemperatur aufgewärmt und der Rückstand zusammen mit dem Inhalt des Reaktionsgefäßes destilliert. Ausbeute 743 T (90,4%) $CF_3-CFJ-COF$. Kp. 61 - 62°C.

## 4) 2-Bromtetrafluorpropionsäurefluorid

Nach der Arbeitsvorschrift des Beispiels 3 wurden 20 T Kaliumfluorid und 100 T Tetraglyme mit 3026 T $CF_3$-$CFBr$-$CF_2$-$OSO_2F$ umgesetzt. Die Destillation ergab 1810 T (86,7 %) $CF_3$-$CFBr$-$COF$. Kp. 29 - 30°C.

## 5) 2-Jodtetrafluorpropionsäuremethylester

In einem Glaskolben, der mit Magnetrührer, Thermometer, Tropfrichter und Rückflußkühler ausgestaltet war, wurden 64 T Methanol vorgelegt und 220 T $CF_3$-$CFJ$-$COF$ unter Eiskühlung zugetropft. Es wurde 30 Minuten bei Raumtemperatur nachgerührt, der Ansatz auf Wasser gegossen und die organische Phase zweimal mit Wasser gewaschen, die nach dem Trocknen über $Na_2SO_4$ durch Destillation gereinigt wurde. Ausbeute: 202 T (88 %) $CF_3$-$CFJ$-$COOCH_3$, Kp. 60 - 62°C/67 mbar.

## 6) 2-Bromtetrafluorpropionsäuremethylester

Nach der Vorschrift des Beispiels 5 wurden 250 T Methanol mit 599 T $CF_3$-$CFBr$-$COF$ umgesetzt. Ausbeute 540 T (85,6 %) $CF_3$-$CFBr$-$COOCH_3$, Kp. 109 - 110°C.

## 7) 2-Bromtetrafluorpropionsäuremethylester aus $CF_3$-$CFBr$-$CF_2$-$OSO_2F$

In der Apparatur des Beispiels 5 wurden 41 T Kaliumfluorid und 250 T Methanol vorgelegt. Dann wurden 228 T eines Gemisches aus 204 T $CF_3$-$CFBr$-$CF_2$-$OSO_2F$ und 24 T $CF_3$-$CFOSO_2$-F-$CF_2OSO_2F$ zugetropft, wobei eine exotherme Reaktion eintrat und Gas entwich. Nach dem Rühren bei Raumtemperatur über Nacht wurde der Ansatz auf 600 T Wasser gegossen und die untere Phase abgetrennt, die noch zweimal mit Wasser gewaschen und über Natriumsulfat getrocknet wurde. Die Destillation lieferte 114,5 T (77%) $CF_3$-$CFBr$-$COOCH_3$.

## 8) Jodtetrafluorpropionsäure-hexafluorisopropylester

In einem 1 000 ml Rührkolben wurden 178 T $CF_3$-$CFJ$-$COF$ vorgelegt. Bei 19°C wurden in 25 Minuten 104 T Hexafluorisopropanol zugetropft. Es wurde auf 0°C abgekühlt und bei dieser Temperatur (Kühlung) in einer Stunde 48,1 T Tributylamin zugegeben. Nach einer weiteren Stunde wurde das Reaktionsgemisch auf Raumtemperatur erwärmt und der Rohester bei 100 mbar abdestilliert. Die anschließende Feindestillation über 30 cm Vigreuxkolonne bei 100 mbar ergab 144 T (52,5 %) $CF_3$-$CFJ$-$COOCH(CF_3)_2$, Kp. 62 - 63°C/ 100 mbar.

## 9) 2-Bromtetrafluorpropionsäurehexafluorisopropylester

In der Apparatur des Beispiels 5 wurden 227 T $CF_3$-$CFBr$-$COF$ vorgelegt. Anschließend wurde ein Gemisch aus Hexafluorisopropanol und 74 T Tributylamin zugetropft. Durch Eiskühlung wurde die Innentemperatur des Gemisches während der gesamten Reaktion kleiner als 22°C gehalten. Anschließend wurde bei 65 - 70 mbar destilliert. Das Rohdestillat und der Falleninhalt wurden erneut destilliert, wobei 296 T (83 %) $CF_3$-$CFBr$-$COOCH$-$(CF_3)_2$ erhalten wurden, Kp. 99 - 101°C.

## 10) Trifluoracrylsäuremethylester a) aus 2-Jodtetrafluorpropionsäuremethylester

In einem Glaskolben, der mit Magnetrührer, Thermometer, Tropftrichter, Vigreuxkolonne, Kolonnenkopf und nachgeschalteter Kältefalle (-78°C) ausgestattet war, wurden 23 T Zinkstaub, 5 T $CF_2$$Br$-$CFClBr$ und 130 T Benzonitril vorgelegt. Das Gemisch wurde erhitzt, bei ca. 110°C setzte Gasentwicklung ($CF_2$=$CFCl$) ein. Nach dieser Aktivierung des Zinkes wurden bei 110°C 71,5 T $CF_3$-$CFJ$-$COOCH_3$ zugetropft, das sich unter exothermer Reaktion umsetzte. Nachdem die Reaktion abgeklungen war, wurden im Vakuum (< 250 mbar) die flüchtigen Bestandteile abgezogen, wobei die Sumpftemperatur auf ca. 150°C erhöht wurde. Destillat und Falleninhalt wurden anschließend fraktioniert, wobei 28 T einer farblosen Flüssigkeit isoliert wurden, Kp. 84 - 88°C, die nach gaschromatographischer Untersuchung zu mehr als 96 % aus $CF_2$=$CF$-$COOCH_3$ bestanden (Ausbeute ca. 80 %). Als Verunreinigung konnte $CF_3$-$CHF$-$COOCH_3$ nachgewiesen werden.

b) aus 2-Bromtetrafluorpropionsäuremethylester

Nach der unter a) beschriebenen Vorschrift wurden 39 T Zinkstaub, 5 T $CF_2Br$-$CFClBr$ und 100 T $CF_3$-$CFBr$-$COOCH_3$ in 150 T Benzonitril umgesetzt. Die Destillation ergab 32,5 T (55,5 %) $CF_2$=$CF$-$COOCH_3$, Kp. 84 - 85°C.

11) Trifluoracrylsäurehexafluorisopropylester

a) In einem 500 ml Rührkolben mit Destillationsbrücke wurden 31,5 T Zinkstaub und 130 T Benzonitril vorgelegt. Bei 135°C wurden zunächst 6,8 T $CF_2Br$-$CFClBr$ eingetropft, wobei eine Gasentwicklung und eine Temperaturerhöhung auf 145°C beobachtet wurden. Nachdem die Temperatur wieder auf 135°C gefallen war, wurden 144 T 2-Jodtetrafluorpropionsäure-hexafluorisopropylester innerhalb 75 Minuten zugetropft (Temp. ~ 135°C), wobei ein Teil des gebildeten Trifluoracrylsäureesters abdestillierte. Nach Ende des Zutropfens ließ man noch 30 Minuten nachreagieren und destillierte dann bei ~ 200 mbar den Rohester ab. Brückendestillat und Rohester wurden vereinigt und über eine 30 cm Vigreuxkolonne destilliert. Man erhielt 50,2 T Trifluoracrylsäurehexafluorisopropylester (53,2 % d. Th.), Kp. 88,5-89°C. Gaschromatographisch wurde als Verunreinigung $CF_3$-$CHF$-$COOCH(CF_3)_2$ nachgewiesen.

b) In einer Apparatur, wie in Beispiel 11 a beschrieben, wurden 19,5 T Zinkstaub und 100 T Benzonitril vorgelegt, auf ca. 130°C erhitzt und zur Aktivierung 50 mg Jod zugegeben wurden. Anschließend wurden 98 T $CF_3$-$CFBr$-$COOCH(CF_3)_2$ zugetropft, wobei nach kurzer Induktionszeit eine exotherme Reaktion einsetzte. Nach beendeter Umsetzung wurde das Produkt durch Destillation abgetrennt und fraktioniert.
Ausbeute 30 T $CF_2 = CF$-$COOCH(CF_3)_2$, Kp. 88 - 89°C, die noch 10 Mol-% $CF_3$-$CHF$-$COOCH(CF_3)_2$ enthalten.

## Ansprüche

1. Verfahren zur Herstellung von Trifluoracrylsäureestern der Formel $CF_2 = CF$-$COOR$ (I), in der R eine verzweigte oder unverzweigte unsubstituierte oder mit Halogen substituierte Alkylgruppe mit 1 bis 6 C-Atomen darstellt, dadurch gekennzeichnet, daß

a) Hexafluorpropen mit einem Halogenfluorosulfat der Formel $X$-$O$-$SO_2$-$F$, in der X Chlor, Brom oder Jod ist, zu $CF_3$-$CFX$-$CF_2OSO_2F$ (II) umgesetzt,

b) aus (II) durch Abspaltung von $SO_2F_2$ das Säurefluorid $CF_3$-$CFX$-$COF$ (III) hergestellt,

c) die Verbindung (III) mit einem geradkettigen oder verzweigten unsubstituierten oder halogensubstituierten aliphatischen Alkohol ROH mit 1 bis 6 C-Atomen im Alkylrest R oder mit einem solchen Alkohol, in dem mindestens ein Wasserstoffatom der Alkylkette mit Ausnahme der α-ständigen H-Atome durch Halogen ersetzt ist, zu $CF_3$-$CFX$-$COOR$ (IV) verestert und

d) der erhaltene Ester (IV) mit einem Metall in einem aprotischen, polaren Lösungsmittel unter Ausbildung einer Doppelbindung zu Verbindung der Formel (I) enthalogeniert wird.

2. Verfahren nach Anspruch 1, dadurch gekennzeichnet, daß R in Formel (I) eine Alkylgruppe mit 1 bis 4 C-Atomen ist und Halogen Fluor bedeutet.

3. Verfahren nach Anspruch 1 oder 2, dadurch gekennzeichnet, daß R in Formel (I) eine Methyl- oder Hexafluorisopropylgruppe ist.

4. verfahren nach einem oder mehreren der Ansprüche 1 bis 3, dadurch gekennzeichnet, daß in der Verfahrensstufe b) mindestens ein lösliches Alkali-oder Erdalkalifluorid oder Trialkylamin mit jeweils 1 bis 6, vorzugsweise 1 bis 4 C-Atomen im Alkylrest als Katalysator eingesetzt wird.

5. Verfahren nach einem oder mehreren der Ansprüche 1 bis 4, dadurch gekennzeichnet, daß in der Verfahrensstufe c) mindestens ein aliphatischer Alkohol mit 1 - 4 C-Atomen oder ein solcher eingesetzt wird, in dem die Wasserstoffatome mit Ausnahme der α-ständigen H-Atome durch Fluor substituiert sind.

6. Verfahren nach einem oder mehreren der Ansprüche 1 bis 5, dadurch gekennzeichnet, daß der Verfahrensschritt d) bei 60 bis 150, vorzugsweise 80 bis 140°C durchgeführt wird.

7. Verfahren nach einem oder mehreren der Ansprüche 1 bis 6, dadurch gekennzeichnet, daß die Stufen b) und c) in einem Schritt durchgeführt werden.

8. Verfahren nach einem oder mehreren der Ansprüche 1 bis 7, dadurch gekennzeichnet, daß als Metall Magnesium, Kupfer oder Zinn, vorzugsweise Zink eingesetzt wird.

9. Tetrafluorpropionsäurefluoralkylester der allgemeinen Formel

$CF_3CFX$-$COO$-$CHRR'$

in der X = Cl, Br, J und
R und R' unabhängig voneinander Wasserstoff oder Per-bzw. Polyfluoralkyl mit 1 - 4 C-Atomen im Alkylrest bedeuten, jedoch R und R' nicht gleichzeitig Wasserstoff sind.

10. Trifluoracrylsäure-1.1.1.3.3.3-hexafluorisopropylester, $CF_2 = CF$-$COO$-$CH(CF_3)_2$.